Europäisches Patentamt

European Patent Office

Office européen des brevets

(11) Veröffentlichungsnummer: **0 356 945 B1**

(19)

(12) **EUROPÄISCHE PATENTSCHRIFT**

(45) Veröffentlichungstag der Patentschrift: **24.11.93**

(21) Anmeldenummer: **89115761.2**

(22) Anmeldetag: **26.08.89**

(51) Int. Cl.⁵: **A61K 37/64**, A61K 35/16

(54) Verwendung von Plasminogen-Aktivator-Inhibitor (PAI-2) zur Immunsuppression.

(30) Priorität: **31.08.88 DE 3829523**

(43) Veröffentlichungstag der Anmeldung:
**07.03.90 Patentblatt 90/10**

(45) Bekanntmachung des Hinweises auf die
Patenterteilung:
**24.11.93 Patentblatt 93/47**

(84) Benannte Vertragsstaaten:
**AT BE CH DE ES FR GB GR IT LI LU NL SE**

(56) Entgegenhaltungen:
**EP-A- 0 208 785**
**EP-A- 0 278 696**
**EP-A- 0 358 995**
**DE-A- 3 713 272**

**THROMBOSIS AND HAEMOSTASIS, Band 56,
Nr. 3, 15. Dezember 1986, Seiten 415-416; Dr.
D. COLLEN: "Report on the meeting of the
subcommittee on fibrinolysis, Jerusalem, Israel, June 2, 1986"**

(73) Patentinhaber: **BEHRINGWERKE Aktiengesellschaft
Postfach 1140
D-35001 Marburg(DE)**

(72) Erfinder: **Stief, Thomas, Dr.
Avda. Kansas City
28 Bloque
1. Apt. 225
E-41007 Sevilla(ES)**
Erfinder: **Heimburger, Norbert, Prof. Dr.
Sonnenhang 10
D-3550 Marburg(DE)**
Erfinder: **Schorlemmer, Hans Ulrich, Dr.
Am Kirschenwald 2
D-3550 Marburg(DE)**

## Beschreibung

Die Erfindung betrifft die Verwendung von Plasminogen-Aktivator-Inhibitor (PAI-2) als Immunsuppressivum.

Makrophagen und polymorphkernige neutrophile Leukozyten (PMN) sind an der Immunantwort des Organismus in wesentlicher Weise beteiligt. Sie verfügen über ein Angriffssystem, das zum einen aus Proteasen und zum anderen aus potenten Oxidantien (N-Chloramine und Sauerstoffradikale) besteht. Über diese Proteasen und Oxidantien beeinflussen und verstärken sie die Immunantwort.

Unter Immunantwort soll verstanden werden die Reaktion immunkompetenter Zellen wie Monozyten/Makrophagen-System auf Entzündungs-, Infektions-, Gewebeumbau- und Gewebereparatur-Reize. Dem Monozyten/Makrophagen-System kommen vielfältige Aufgaben im gesamten Organismus zu, da Blutmonozyten in verschiedenste Gewebe einwandern, um hier nicht zuletzt spezifische immunologische Aufgaben zu übernehmen. So differenzieren sich die Monozyten in der Lunge zu Alveolarmakrophagen, in der Leber zu von Kupfferschen Sternzellen, im Knochen zu Osteoklasten, im zentralen Nervensystem (ZNS) zu Mikrogliazellen, in der Niere zu Mesangialmakrophagen, in der Gelenkschleimhaut zu Synovialisdeckzellen und in den Körperhöhlen zu Pleura- bzw. Peritonealmakrophagen.

Inhibitoren dieser Makrophagen und PMN sind von hohem klinischen Interesse, da eine Vielzahl menschlicher Erkrankungen mit erhöhter Makrophagenaktivität einhergeht.

Es kann Zustände geben, in denen die funktionelle Unterdrückung eines oder mehrerer Kompartimente des Immunsystems wünschenswert ist. Solche Zustände können eine über das Normalmaß hinausschießende Reaktion des Immunsystems (hypererger Zustand) oder eine Reaktion des Immunsystems gegen körpereigenes Gewebe sein.

Substanzen, die in der Lage sind, die biologische Wirkung von Makrophagen zu inhibieren, können immunsuppressiv wirken. Es besteht großer Bedarf nach a) nebenwirkungsarmen und b) hochspezifischen Immunsuppressoren. Die in der Klinik vielfach verwandten Kortikoidabkömmlinge erfüllen weder a) noch b).

Überraschenderweise wurde gefunden, daß in in vitro-Testsystemen mit Makrophagen und deren Sekretionsprodukten Plasminogen-Aktivator-Inhibitor (PAI-2) eine Sauerstoffradikal-Sekretion inhibierende Wirkung zeigt.

Nach Thrombosis and Haemostasis 56, 415-416 (1986) hat PAI-2 unter anderem folgende Eigenschaften: Molekulargewicht 47 000 (unglycolysiert), 60 000 (glycosyliert); isoelektrischer Punkt 5 (4,4); Inhibitionskonstante für Urokinase: $9 \cdot 10^5 \, M^{-1} s^{-1}$.

In EP-A-0 278 696 ist eine gentechnische Herstellung von PAI-2 beschrieben.

Überraschend ist, daß PAI-2 Urokinase in oxidativer Umgebung zu inhibieren vermag, d. h. PAI-2 ist ein oxidationsresistenter PAI, im Gegensatz zu PAI vom endotheliären Typ (PAI-1).

Es wurde überraschenderweise gefunden, daß PAI-2 in physiologischen Konzentrationen stark inhibierend auf die auf Sauerstoffradikal-Freisetzung beruhende Chemilumineszenzreaktion von Makrophagen wirkt. Damit einhergehend wurde gefunden, daß PAI-2 in vivo an Transplantationsmodellen immunsuppressiv wirkt.

Gegenstand der Erfindung ist deshalb die Verwendung des placentären Plasminogen-Aktivator-Inhibitors (PAI-2) in einem Verfahren zur Herstellung eines Mittels zur Immunsuppression. Krankheiten, bei denen ein solches Mittel angewendet werden kann, sind beispielsweise Autoimmunerkrankungen wie Psoriasis, Glomerulonephritis, vorzugsweise der Mesangialfelder, Multipler Sklerose, Guillan-Barré-Syndrom, Kollagenosen, allergischen Erkrankungen, Schönlein-Henloch-Syndrom, degenerativen Erkrankungen wie Morbus Parkinson, Arteriosklerose, Wunde- und Knochenheilungs- und -umbaustörungen oder Schocksyndrom wie ARDS (Adult Respiratory Distress Syndrome) Verbrennungskrankheit und Verbrauchskoagulopathie. Auch bei infektiösen Erkrankungen wie AIDS könnte einem solchen Mittel Bedeutung zukommen.

Auch nach der Transplantation von körperfremdem Gewebe kommt es zu einer immunologischen Reaktion gegen das als fremd erkannte transplantierte Organ oder gegen den Empfängerorganismus im Sinne einer graft versus host-Reaktion, was zu einer Abstoßung führt. Derartige Abstoßungskrisen können sich auch mit plazentarem Gewebe ergeben, welches vom mütterlichem Organismus z. T. auch als fremd erkannt wird und zu Plazentathrophoblaststörungen und vorzeitiger Plazentalösung führen kann. Es ist daher notwendig, das Immunsystem des Organempfängers oder des Transplantats zu unterdrücken, um das Überleben des Transplantats bzw. des Empfängers zu gewährleisten. Komplikationen (Nachblutungen) nach erfolgter Subarachnoidalblutung sind ebenfalls mitbedingt durch erhöhte Aktivität weißer Blutzellen.

Im Gegensatz zu der in der Klinik bei Erkrankungen wie Morbus Crohn, Colitis ulcerosa, Kollagenosen oder Multiple Sklerose eingesetzten Superoxiddismutase, welche entstandene Sauerstoffradikale zu neutralisieren vermag, verhindert PAI-2 die Entstehung von Sauerstoffradikalen. PAI-2 zeigt auch Wirkung bei allergischer Encephalomyelitis (AE). AE gilt als Modell für die humane Erkrankung Multiple Sklerose.

Über die Norm hinausschießende Reaktionen des Immunsystems und damit einhergehende pathologisch erhöhte Sauerstoffradikal-Freisetzung findet sich auch bei Reperfusionszuständen, d. h. plötzliche Blutdurchströmung eines zuvor minderperfundierten Gewebes, bei hochprozentiger Sauerstoffbeatmung, bei klinischem Einsatz bzw. Vergiftung mit z. B. Paraquat, Anwendung von Cis-Platin, Adriamycin, Nitrofurantoin, Bleomycin, Streptozotocin und anderen diabetogenen Substanzen, Bestrahlungstherapie und damit einhergehenden Gewebeschäden.

Zu den bekannten Maßnahmen, die für die Unterdrückung des Immunsystems geeignet sind, gehören die Behandlung mit Antikörpern gegen lymphatisches Gewebe, ionisierenden Strahlen und chemischen Substanzen. Eine derartige aggressive Behandlung geht einher mit ausgeprägten Nebenwirkungen: Organotoxizität, Sterilität bei Cytostatika und ionisierenden Strahlen, Hirnödem bei Tranexamsäure, Anti-Antikörperbildung mit Gefahr einer Serumkrankheit bei Behandlung mit Antikörpern.

Verwendung des humanen physiologischen PAI hat dagegen keine der Nebenwirkungen und wirkt bereits in niedrigen

Dosen wesentlich effektiver. Selbst hohe Konzentrationen von PAI-2 im Plasma von z. B. gesunden Schwangeren sind beschrieben worden. Daraus ergibt sich eine hohe therapeutische Breite dieses Proteins mit mindestens bis zu 30 Urokinase inhibierenden Einheiten/ml Plasma (200 - 300 ng/ml).

Eine wirksame Menge an PAI-2 zur Immunsuppression und Inhibition von Sauerstoffradikalfreisetzung, z. B. zum Inhibieren des Schadens bei gefährdetem Gewebe während der Reperfusion, Subarachnoidalblutungen, Autoimmunerkrankungen, multipler Sklerose, Kollagenosen, allergischen Erkrankungen, Schönlein-Henoch-Syndrom, degenerativen Erkrankungen, Arteriosklerose, Wund-und Knochenheilungs- und -umbaustörungen, Verbrennungskrankheiten, Transplantatunverträglichkeit, vorzeitiger Plazentalösung und Präeklampsie, Schocksyndrom hängt von einer Reihe von Faktoren ab, beispielsweise dem Alter und dem Gewicht des Patienten und dem klinischen Zustand.

Eine wirksame DOSIS liegt im allgemeinen im Bereich von 0.7 - 30 000 und vorzugsweise 7 - 3000 Urokinase-inhibierenden Einheiten/kg/24 h, d.h. 525 - 225 000 Einheiten, applizierbar i. v. oder lösungsvermittelt i. m. für einen 75 kg schweren Patienten. PAI-2 kann durch Zusatz von Stabilisatoren wie Albumin, Polygelin oder Glycin stabilisiert werden.

PAI-2 kann auch topisch verabreicht werden zur lokalen Therapie und Prophylaxe von Wundheilungsstörungen, Verbrennungen, Transplantatabstoßungen, allergischen Respirationserkrankungen und anderen mit erhöhter Makrophagenaktivität einhergehenden Erkrankungen.

Medizinisch indiziert sind Kombinationen aus PAI-2 und einem Fibrinolytikum wie Gewebe-Plasminogenaktivator (t-PA), Urokinase, Streptokinase oder Plasminogen-Strep-tokinase-Aktivatorkomplex zur Lysetherapie vorzugsweise bei arteriellen Verschlüssen wie bei Myokardinfarkt oder Apoplex. Ein kombinierter Einsatz von PAI-2 und Fibrinolytikum ermöglicht die sofortige Inhibition von Gewebeschäden, wie Z.B. Nekrosen während und nach akuter Reperfusion bedingt durch hyperaktive weiße Blutzellen. Wird in Kombination mit PAI-2 als Fibrinolytikum einer der natürlichen Plasminogenaktivatoren t-PA oder Urokinase verwendet, finden vorzugsweise die einkettigen Formen dieser Substanzen ihren Einsatz, da sie, im Gegensatz zu den Zweikettenformen, keine oder so gut wie keine inaktiven Komplexe mit PAI-2 bilden.

Die wirksame Dosis an Fibrinolytikum in Kombination mit PAI-2 liegt bei 1 - 200 mg t-PA oder Urokinase bzw. 100.000 - 3 Mio. Einheiten Streptokinase oder Aktivatorkomplex für die systemische Applikation bei einem 75 kg schweren Patienten.

Weiterhin medizinisch angezeigt sind Kombinationen aus PAI-2 und einem Ornithindecarboxylase-Inhibitor niedermolekularer wie einem Derivat eines Polyamines oder hochmolekularer wie einer Transglutaminase Art. Besonders bevorzugt ist die Kombination von PAI-2 mit einer Transglutaminase wie F XIII, plazentaren, plasmatischen oder gentechnologischen Ursprungs zur Verwendung als Immunsuppressivum. Wird F XIII in einer Kombination mit PAI-2 verwendet, dann beträgt eine wirksame Dosis an F XIII im allgemeinen 0.7 - 300 E/kg/24 h für die systemische Applikation bei einem 75 kg schweren Patienten.

Im folgenden wird die Wirkung der erfindungsgemäßen Substanzen auf die Immunantwort der Maus und des Menschen in ausgewählten in vivo- und in vitro-Standardtestmethoden, die bekanntermaßen für die Beurteilung von Immunsuppressoren herangezogen werden, beispielhaft erläutert.

Beispiel 1

Einfluß von PAI-2 (Urokinase-Inhibitor) auf die Stimulation humaner Phagozyten in vitro

Es wurden polymorphkernige Granulozyten und mononukleäre Phagozyten aus peripherem Blut gesunder Spender gewonnen und nach Gabe des Präparates auf verschiedene Funktionen geprüft. Als Parameter der Phagozytenfunktion wurden die Chemilumineszenzreaktion (Sauerstoff-Radikalbildung) und die Sekretion lysosomaler Enzyme untersucht. Gemessen wurde sowohl die Wirkung von PAI-2 auf die nicht-aktivierten (-IC) als auch auf die durch Immunkomplexe aktivierten Phagozyten (+IC). PAI-2 wurde nach J. Biol. Chem. 262, 3646-3653 (1987) gewonnen.

Verglichen mit Phagozyten der entsprechenden Kontrollgruppen (unbehandelte Zellen) war die Chemilumineszenzreaktion (Generierung von $O_2$-Radikalen) sowohl bei humanen Granulozyten als auch Monozyten (Phagozyten) dosisabhängig signifikant erniedrigt (Tabelle 1).

Beispiel 2

Wirkung von PAI-2 auf die Aktivität von Maus-Peritonealmakrophagenin vivo

Weiblichen NMRI Mäuse (18 - 20 g) wurde Urokinase-Inhibitor (PAI-2) in verschiedenen Konzentrationen von 5 - 100 E/Tier intraperitoneal verabreicht. Die Kontrollen erhielten gleiche Volumina (0,5 ml) des Lösungsmittels (physiologisch gepufferte Kochsalzlösung, pH 7,2). Zwei Stunden und 24 Stunden später wurden die Mäuse getötet, ihnen die Makrophagen aus der Bauchhöhle entnommen und eine Aktivitätsbestimmung mit Hilfe der Chemilumineszenz, wie im Beispiel 1 beschrieben, durchgeführt. Wie aus Tabelle 2 ersichtlich ist, erniedrigt PAI-2 die Aktivität von Makrophagen, die Mäusen entnommen wurden, welche zuvor mit dem Präparat behandelt wurden. Die Suppression wurde bei der Chemilumineszenz sowohl nach 2 Stunden als auch nach 24 Stunden nach Applikation der Substanz mit und ohne Zusatz von Immunkomplexen in vitro beobachtet.

Beispiel 3

Einfluß von Chloramin T-Oxidation auf die Aktivität von Plasminogen-Aktivator-Inhibitor (PAI) in Plasma

a) unterschiedliche Chloramin T-Konzentration 50 $\mu$l PAI-Mangelplasma (Biopool, Umea, Schweden), PAI-1 reiches Plasma (15 Urokinase-inhibierende Einheiten (E/ml)) sowie PAI-Mangelplasma supplementiert mit 15 E PAI-2/ml wurden mit 50 $\mu$l 100 mmol/l Tris, 100 mmol/l NaCl, 1 % [R]Haemaccel, 0,1 % [R]Triton x 100-Puffer (TNHT), pH 8,5, und 50 $\mu$l Chloramin T (CT) unterschiedlicher Konzentrationen in aqua dest. 10 min bei Raumtemperatur inkubiert. Danach wurden 200 $\mu$l TNHT-Puffer mit 5 IU Urokinase (u-PA) und zum Nullabgleich mit 0 IU u-PA/ml zugesetzt und weitere 10 min bei Raumtemperatur inkubiert, um u-PA-PAI-Komplexe zu erzeugen. Anschließend wurde die verbleibende u-PA-Aktivität mit dem von Stief et al. beschriebenen Verfahren (Thromb. Res. 50: 559-573, 1988) bestimmt, indem 200 $\mu$l 10 mmol/l Chloramin T, 3 mmol/l Tranexamsäure in aqua dest. zugefügt wurden, 15 min bei Raumtemperatur inkubiert wurde und die Nachweisreaktion durch Zusatz von 500 $\mu$l 0,6 mmol/l chromogenem Plasminsubstrat HD-Nva-CHA-Lys-pNA (pNA = para Nitroanilide) in 480 mmol/l Nacl gestartet wurde.

Nach 10 minütiger Inkubation bei Raumtemperatur wurde die Substratreaktion durch Zusatz von 100 $\mu$l 8,4 mol/l Essigsäure abgestoppt und die Extinktion bei 405 nm bestimmt. Ergebnis siehe Tabelle 3.

Bereits ab 0,1 $\mu$mol CT treten PAI-1-Aktivitätsverluste ein, während PAI-2 ca. 5fach resistenter gegenüber CT-Oxidation ist.

0,5 $\mu$mol CT bewirkt in 50 $\mu$l Plasma bereits über 50 % Verlust an aktivem PAI-1, während dieselbe Dosis CT noch 77 % der PAI-2-Aktivität intakt läßt.

b) Einfluß unterschiedlicher PAI-2, [R]Fibrogammin und Ascorbinsäure-Konzentrationen auf oxidativ verursachten Kontrollverlust von Plasma über Plasminogen-Aktivator-Aktivität

100 $\mu$l Standard-Human-Plasma, citratantikoaguliert, wurde mit 50 $\mu$l a) PAI-2, b) [R]Fibrogammin c) Ascorbinsäurelösung unterschiedlicher Konzentration in aqua dest. mit 250 $\mu$l TNHT-Puffer mit 1,25 IU u-PA, 100 $\mu$l 10 mmol/l CT und 100 $\mu$l 3 mmol/l HD-Nva-CHA-Lys-pNA 60 min bei 37 °C inkubiert. Darauf wurde die Substratumsetzung durch Zusatz von 500 $\mu$l 3,4 mol/l Essigsäure beendet und die resultierende Extinktion bei 405 nm bestimmt. Ergebnis siehe Tabelle 4.

Man erkennt, daß PAI-2 (10 E) sowie [R]Fibrogammin (0,5 Fibrogammin-Einheiten) sowie Vitamin C (20 $\mu$mol) zu einer Wiederherstellung des Kontrollverlustes über das u-PA/ Plasmin-System führen. Die anfänglich erhöhten Extinktionen nehmen bei Supplementieren des Plasmas mit den Substanzen bis auf 0 % ab. Vitamin C kann durch N-acetylcystein oder N-acetylmethionin ersetzt werden.

Beispiel 4

Effekt verschiedener CT-Konzentrationen auf u-PA/Plasminogen/Plasmin-System, Neutralisation des Effektes durch PAI-2 und/oder Antagosan in Kombination mit Vitamin C (Ascorbinsäure)

100 $\mu$l Standard-Human-Plasma, antikoaguliert mit EDTA, werden mit 250 $\mu$l TNHT-Puffer, enthaltend 1,25 IU u-PA, und 100 $\mu$l aqua dest., enthaltend a) 10 mmol/l Ascorbinsäure, b) 2,5 E PAI-2, c) 10 mmol/l Ascorbinsäure, 2,5 E PAI-2, d) 2,5 KIU[R]Antagosan, e) 2,5 KIU[R]Antagosan, 2,5 E PAI-2, f) 2,5 KIU[R]Antagosan, 10 mmol/l Ascorbinsäure, 2,5 E PAI-2, h) (Kontrolle) versetzt. Daraufhin wurde 100 $\mu$l CT unterschiedlicher Konzentration in aqua dest. zugefügt und 45 min bei 37 °C inkubiert. Zusatz von 500 $\mu$l 0,6 mmol/l HD-Nva-CHA-Lys-pNA in 480 mmol/l NaCl startete die Substratreaktion. Nach 6 min (37 °C) wurde die Substratumsetzung gestoppt durch Zusatz von 500 $\mu$l 20 % Essigsäure. Ergebnis siehe Tabelle 5.

Man erkennt, daß steigende Mengen an CT zu proportional steigender Extinktionsmessung bei 405 nm führt. Die physiologische Plasmakontrolle über die Serinproteaseaktivität von Plasmin und Urokinase ist verlorengegangen. Durch Supplementieren von Humanplasma mit [R]Antagosan und/oder PAI-2 kann die Kontrolle wiederhergestellt und wesentlich weniger Plasminaktivität nachgewiesen werden. Der Einsatz von Vitamin C verstärkt den Effekt von PAI-2/[R]Antagosan in synergistischer Weise.

Beispiel 5

Einfluß von PAI-2 auf die Überlebenszeit von transplantierter Rattenschwanzhaut

In diesem Experimentalansatz wurden Schwanzhautstückchen von Lewis-Ratten mit einer Größe von etwa 0,5 x 1,0 cm auf den Schwanz von Fischer-Ratten verpflanzt. Die transplantierten Hautstücke werden vom Immunsystem der Empfängertiere als fremd erkannt und abgestoßen. Wie in der Tabelle 6 zu sehen ist, betrug die Transplantatüberlebenszeit im Lewis-Fischer-Rattenmodell in den Kontrollgruppen, die nur mit dem Lösungsmittel behandelt wurden, zwischen 16 und 18 Tagen. PAI-2 (50 E/Tier pro Injektion) wurde intraperitoneal an 7 aufeinanderfolgenden Tagen entweder beginnend am Tag 1 oder am Tag 10 nach Transplantation verabreicht. Hierbei zeigte sich überraschend, daß PAI-2 in der benutzten Konzentration die Überlebenszeiten der Transplantate von 17,0 ± 1,4 auf 26,0 ± 2,0 bzw. 23,0 ± 2,4 erhöhte.

Tabelle 1

| Einfluß von PAI-2 auf den oxidativen Stoffwechsel (Chemilumineszenz) mit und ohne Immunkomplexstimulation (50 $\mu$g/ml) in vitro | | |
|---|---|---|
| Zelltyp | PAI-2 (E/ml) | Chemilumineszenz Integral der RLU/15 min.(x10$^3$) |
| | | -IC / + IC |
| Human-Monozyten (Phagozyten) 1x10$^6$ Zellen | 0 | 2478 ± 354 / 18516 ± 1571 |
| | 0.07 | 1321 ± 92 / 9347 ± 709 |
| | 0.15 | 1061 ± 35 / 4825 ± 403 |
| | 0.7 | 811 ± 68 / 2640 ± 235 |
| | 1.5 | 633 ± 49 / 1580 ± 221 |
| | 6.0 | 452 ± 31 / 1040 ± 163 |
| | 25.0 | 247 + 75 / 695 + 125 |
| Human-Granulozyten 2x10$^5$ Zellen | 0 | 3110 ± 127 / 48547 ± 2372 |
| | 0.05 | 1656 ± 252 / 28633 ± 4394 |
| | 0.1 | 1172 ± 131 / 15567 ± 1762 |
| | 0.5 | 1018 ± 94 / 12267 ± 709 |
| | 2.5 | 422 ± 48 / 6587 ± 604 |
| | 5.0 | 135 ± 22 / 4550 ± 665 |

Tabelle 2

| Einfluß von PAI-2 (Urokinase-Inhibitor) auf die Makrophagenaktivität (1 x $10^6$ Zellen) in vivo. | | | | |
|---|---|---|---|---|
| PAI-2 E/Tier 1 x i.p. | Chemilumineszenz Integral der RLU/15 min. (x $10^3$) | | | |
| | -IC | | + + IC | |
| | 2 Std. | 24 Std. | 2 Std. | 24 Std. |
| 0 | 2775 ± 375 | 4480 ± 453 | 51800 ± 1286 | 72500 ± 1556 |
| 5 | 1655 ± 106 | 3440 ± 142 | 27175 ± 1859 | 23950 ± 1625 |
| 25 | 1145 ± 70 | 1467 ± 85 | 19267 ± 1550 | 17200 ± 1407 |
| 50 | 600 ± 118 | 915 ± 19 | 13833 ± 2346 | 8953 ± 287 |
| 100 | 466 ± 75 | 488 ± 166 | 9783 ± 2188 | 4960 ± 392 |

## Tabelle 3

| CT-Dosis ($\mu$mol/50$\mu$l) | Extinktion MPL-PAI PAI-1 PAI-2 ($A_{405nm}$ x 1000) | | | Hemmung von u-PA * PAI-1 PAI-2 PAI-1 PAI-2 (%) (%) ** | | | |
|---|---|---|---|---|---|---|---|
| 0 | 1011 | 350 | 485 | 65 | 52 | 100 | 100 |
| 0,01 | 1016 | 377 | 498 | 63 | 51 | 97 | 98 |
| 0,05 | 1033 | 351 | 503 | 66 | 51 | 101 | 98 |
| 0,1 | 1050 | 459 | 509 | 56 | 52 | 86 | 100 |
| 0,175 | 1063 | 541 | 528 | 49 | 50 | 75 | 96 |
| 0,25 | 1099 | 596 | 549 | 43 | 47 | 66 | 90 |
| 0,375 | 1060 | 668 | 593 | 37 | 44 | 57 | 85 |
| 0,5 | 1049 | 735 | 626 | 30 | 40 | 46 | 77 |
| 0,75 | 1001 | 777 | 696 | 22 | 31 | 34 | 60 |
| 1,0 | 982 | 807 | 716 | 18 | 27 | 27 | 52 |
| 1,5 | 921 | 792 | 718 | 14 | 22 | 21 | 42 |
| 2,0 | 834 | 738 | 667 | 11 | 20 | 17 | 38 |

$$* \quad (1 - \frac{\text{Extinktion PAI-Plasma}}{\text{Extinktion PAI-Mangelplasma}}) \text{ X } 100$$

** in % von 0 $\mu$mol CT = aqua dest. Kontrolle

Tabelle 4

| Zusatz von u-PA inhib.E. | PAI-2 | | [R]Fibrogammin | | Vitamin C |
|---|---|---|---|---|---|
| | $A_{405nm}$ (x 1000) | Einh. | $A_{405nm}$ (x 1000) | mmol/l | $A_{405nm}$ (x 1000) |
| 0 | 579 | 0 | 579 | 0 | 579 |
| 1 | 467 | 0,1 | 325 | 5 | 363 |
| 2,5 | 343 | 0,25 | 168 | 10 | 219 |
| 5 | 144 | 0,5 | 63 | 20 | 112 |
| 10 | 61 | 1 | 20 | 30 | 75 |
| 20 | 0,0 | 2 | 25 | 40 | 62 |
| 40 | 0,0 | 4 | 9 | 60 | 50 |
| 80 | 0,0 | 8 | 0,0 | 80 | 36 |

Tabelle 5

| Zusatz CT (mmol/l) | Kontrolle ($A_{405nm}$x 1000) | Generierte Plasminaktivität (% von Kontrolle) | | | | | | |
|---|---|---|---|---|---|---|---|---|
| | h) | a) | b) | c) | d) | e) | f) | g) |
| 0 | 0,0 | - | - | - | - | - | - | - |
| 5 | 32 | 56 | 53 | 65 | 40 | 36 | 37 | 22 |
| 10 | 121 | 30 | 48 | 60 | 26 | 22 | 26 | 9 |
| 20 | 456 | 33 | 50 | 64 | 16 | 21 | 32 | 11 |
| 30 | 870 | 54 | 43 | 62 | 10 | 35 | 29 | 15 |
| 40 | 837 | 100 | 48 | 60 | 47 | 62 | 27 | 29 |

Tabelle 6

| Effekt von PAI-2 auf die Hauttransplantation bei Fischer-Ratten | | |
|---|---|---|
| Substanz | Tage der Transplantatabstoßung | Mittelwert (Tage) (x ± s) |
| Kontrolle | 19, 18, 16, 16, 16 | 17,0 ± 1,4 |
| PAI-2 50 E/Tier 7 x i.p. day 1-7 | 29, 27, 24, 26, 24 | 26,0 ± 2,0 |
| PAI-2 50 E/Tier 7 x i.p. day 10-17 | 22, 27, 24, 22, 21 | 23,2 ± 2,4 |

**Patentansprüche**

1. Verwendung von Plasminogen-Aktivator-Inhibitor (PAI-2) in einem Verfahren zur Herstellung eines Mittels mit immunsuppressiver Wirkung.

2. Verwendung nach Anspruch 1, dadurch gekennzeichnet, daß das Mittel 52 bis 2,25 Mio Urokinase-inhibierende Einheiten pro Dosis (75 kg Körpergewicht) Plasminogen-Aktivator-Inhibitor-2 enthält.

3. Verwendung nach Anspruch 1, dadurch gekennzeichnet, daß das Mittel einen Stabilisator, vorzugsweise Albumin oder abgebaute, vernetzte Gelatine oder abgebautes, vernetztes Kollagen enthält.

4. Verwendung nach Anspruch 1, dadurch gekennzeichnet, daß ein Mittel für die Therapie und Prophylaxe von Transplantat- und Plazentaabstoßungskrisen und Graft versus host-Reaktionen hergestellt wird.

5. Verwendung nach Anspruch 1, dadurch gekennzeichnet, daß ein Mittel für die Therapie von Autoimmunerkrankungen hergestellt wird.

**6.** Verwendung nach Anspruch 1, dadurch gekennzeichnet, daß ein Mittel für die Therapie und Prophylaxe von Wund- und Knochenheilungsstörungen hergestellt wird.

**7.** Verwendung nach Anspruch 1, dadurch gekennzeichnet, daß ein Mittel für die Therapie von Krankheitsprozessen, die mit erhöhter Sauerstoff-Radikalbildung einhergehen, hergestellt wird.

**8.** Verwendung nach Anspruch 1, dadurch gekennzeichnet, daß ein Mittel für die Therapie und Prophylaxe von Gewebeschäden während und nach akuter Reperfusion hergestellt wird.

**9.** Verwendung nach Anspruch 1, dadurch gekennzeichnet, daß ein Mittel für die Prophylaxe und Therapie von Nachblutungskomplikationen bei Subarachnoidalblutung hergestellt wird.

**10.** Verwendung nach Anspruch 1, dadurch gekennzeichnet, daß ein Mittel zur topischen Anwendung, vorzugsweise zur Fibrinklebung, als Wundheilungsspray oder Asthmaspray oder Verbrennungsspray oder zur Therapie und Prophylaxe von mit erhöhter Leukozytenaktivität einhergehenden Erkrankungen hergestellt wird.

**11.** Verwendung nach Anspruch 1, dadurch gekennzeichnet, daß ein Mittel zur Unterdrückung des Monozyten-Makrophagen-Systems hergestellt wird.

**Claims**

**1.** The use of plasminogen activator inhibitor (PAI-2) in a process for the preparation of an agent having an immunosuppressant action.

**2.** The use as claimed in claim 1, wherein the agent contains 52 to 2.25 million urokinase-inhibiting units per dose (75 kg body weight) of plasminogen activator inhibitor 2.

**3.** The use as claimed in claim 1, wherein the agent contains a stabilizer, preferably albumin or degraded crosslinked gelatin or degraded crosslinked collagen.

**4.** The use as claimed in claim 1, wherein an agent is prepared for the therapy and prophylaxis of transplant-and placenta-rejection crises and graft-versus-host reactions.

**5.** The use as claimed in claim 1, wherein an agent is prepared for the therapy of autoimmune diseases.

**6.** The use as claimed in claim 1, wherein an agent is prepared for the therapy and prophylaxis of disturbances of wound and bone healing.

**7.** The use as claimed in claim 1, wherein an agent is prepared for the therapy of pathological processes which are associated with increased formation of oxygen radicals.

**8.** The use as claimed in claim 1, wherein an agent is prepared for the therapy and prophylaxis of tissue damage during and after acute reperfusion.

**9.** The use as claimed in claim 1, wherein an agent is prepared for the prophylaxis and therapy of after-bleed complications of subarachnoid hemorrhage.

**10.** The use as claimed in claim 1, wherein an agent is prepared for topical administration, preferably as a fibrin adhesive, as a wound-healing spray or asthma spray or burn spray or for the therapy and prophylaxis of disorders associated with increased leukocyte activity.

**11.** The use as claimed in claim 1, wherein an agent is prepared for suppressing the monocyte/macrophage system.

**Revendications**

1.  Utilisation d'un inhibiteur de l'activateur du plasminogène (PAI-2) dans un procédé pour la préparation d'un agent à effet immunosuppresseur.

2.  Utilisation selon la revendication 1, caractérisée en ce que l'agent contient de 52 à 2,25 millions d'unités inhibitrices de l'urokinase par dose (75 kg de poids corporel) d'inhibiteur de l'activateur du plasminogène 2.

3.  Utilisation selon la revendication 1, caractérisée en ce que l'agent contient un stabilisant, de préférence de l'albumine ou de la gélatine réticulée dégradée, ou du collagène réticulé dégradé.

4.  Utilisation selon la revendication 1, caractérisée en ce que l'on prépare un agent pour le traitement et la prophylaxie de phénomènes de rejet de greffe et de placenta et de réactions du greffon contre l'hôte.

5.  Utilisation selon la revendication 1, caractérisée en ce que l'on prépare un agent pour le traitement de maladies auto-immunes.

6.  Utilisation selon la revendication 1, caractérisée en ce que l'on prépare un agent pour le traitement et la prophylaxie de troubles de la consolidation osseuse et de la cicatrisation.

7.  Utilisation selon la revendication 1, caractérisée en ce que l'on prépare un agent pour le traitement de processus pathologiques qui apparaissent avec une formation accrue de radicaux oxygénés.

8.  Utilisation selon la revendication 1, caractérisée en ce que l'on prépare un agent pour le traitement et la prophylaxie de lésions tissulaires pendant et après reperfusion intense.

9.  Utilisation selon la revendication 1, caractérisée en ce que l'on prépare un agent pour la prophylaxie et le traitement de complications d'hémorragies secondaires dans l'hémorragie sousarachnoïdienne.

10. Utilisation selon la revendication 1, caractérisée en ce que l'on prépare un agent pour l'administration locale, de préférence pour l'agglutination de la fibrine, sous forme d'aérosol pour cicatrisation ou d'aérosol contre l'asthme ou d'aérosol contre les brûlures ou pour le traitement et la prophylaxie de maladies apparaissant avec une activité leucocytaire accrue.

11. Utilisation selon la revendication 1, caractérisée en ce que l'on prépare un agent pour réprimer le système des macrophages-monocytes.